# EUROPÄISCHE PATENTANMELDUNG

(11) **EP 4 776 499 A1**
(43) Veröffentlichungstag der Anmeldung: **15.07.2026**
(21) Anmeldenummer: 25151151.5
(22) Anmeldetag: 10.01.2025
(51) Int. Cl.: H02M 1/42, H02M 1/00, A61B 18/12

(54) **GENERATOR ZUM BETRIEB CHIRURGISCHER INSTRUMENTE**

(71) Anmelder: Erbe Elektromedizin GmbH, 72072 Tübingen (DE)
(72) Erfinder: EGLER, Thomas, 72770 Reutlingen (DE); RIPPLINGER, Thomas, 72810 Gomaringen (DE)
(74) Vertreter: Rüger Abel Patentanwälte PartGmbB

(57) **Zusammenfassung**

Ein erfindungsgemäßer für gepulsten Betrieb geeigneter Generator (11) weist eine Leistungsfaktor-Korrekturschaltung (21) mit einer integrierten Steuerschaltung (31) auf. Dem Spannungsfühlereingang (VSENSE) der Steuerschaltung (31) ist ein Verstärker (35) vorgeschaltet. Es ist möglich, den Verstärker (35) so auszubilden, dass sein Verstärkungsfaktor in einem ersten Zustand den Wert 1 und in einem zweiten Zustand einen Wert von größer als 1 einnimmt. Weiter ist es möglich, die Verstärkung lediglich dann auf den Wert von größer als 1 zu setzen, wenn die Spannung an dem Wandlerausgang innerhalb eines Toleranzbereichs liegt, der für den gewöhnlichen Betrieb der Leistungsfaktor-Korrekturschaltung (21) vorgesehen ist. Außerhalb dieses Toleranzbereichs ist die Verstärkung des Verstärkers (35) dann genau 1. Damit wird der gewöhnliche Betrieb der integrierten Steuerschaltung 31 insbesondere beim Hochlaufen nicht gestört.

## Beschreibung

Die Erfindung betrifft einen Generator zum Betrieb eines oder mehrerer chirurgischer Instrumente, insbesondere zur Versorgung solcher Instrumente mit elektrischer Leistung.

Elektrochirurgische Instrumente zum Einsatz an menschlichen oder tierischen Patienten sind grundsätzlich aus dem Stand der Technik ebenso bekannt wie Generatoren zur Versorgung solcher Instrumente. Dazu offenbart die EP 2 853 217 B1 einen Generator, an dem ein monopolares Instrument sowie eine zugehörige Neutralelektrode angeschlossen sind. Zur Energieversorgung ist der Generator an das öffentliche Stromnetz anschließbar.

Der Generator weist eingangsseitig einen Netzgleichrichter auf, dem eine Leistungsfaktor-Korrekturschaltung nachgeschaltet ist. Diese ist als Sperrwandler ausgebildet und lädt einen Speicherkondensator auf eine Spannung auf, die über der Spitzenspannung der höchst anzunehmenden Netzspannung liegt. Dem Speicherkondensator kommt dabei die Aufgabe zu, die notwendige Energie zu speichern, um auch bei pulsierenden Lasten einen zu großen Spannungseinbruch, jedenfalls aber einen Spannungseinbruch unter die Netzspitzenspannung zu verhindern, damit kein unkontrollierter Stromfluss durch den Netzgleichrichter und den Sperrwandler auf den Speicherkondensator auftritt. An den Sperrwandler ist ein Gleichspannungswandler angeschlossen, der einen Wechselrichter und einen Transformator zur sicheren Potentialtrennung zwischen patientenseitiger Elektrik und netzseitiger Elektrik bewirkt. Auch der Gleichspannungswandler enthält gesteuerte Schalter und Pufferkondensatoren. Sowohl die Leistungsfaktor-Korrekturschaltung als auch der Gleichspannungswandler weisen Steuerungen auf, die untereinander über eine Datenschnittstelle kommunizieren. An den Gleichspannungswandler ist ein Hochfrequenzoszillator angeschlossen, der die zur Speisung eines chirurgischen Instruments erforderliche hochfrequente Behandlungsspannung bereitstellt.

Zur Realisierung von Leistungsfaktor-Korrekturschaltungen sind integrierte Schaltkreise verfügbar, wie z.B. der ICE3PCS01-DS von Infineon Technologies, dessen Eigenschaften und Anwendungsempfehlungen aus https://www.infineon.com/dgdl/Infineon-ICE3PCS01-DS-v03 00-EN.pdf?fileId=db3a304329a0f6ee0129a67ae8c02b46 ersichtlich sind.

Bei wechselnden Lasten treten auf dem Speicherkondensator der Leistungsfaktor-Korrekturschaltung Spannungsschwankungen auf, deren Begrenzung eine entsprechend großzügige Dimensionierung des Speicherkondensators erfordert. Daraus resultieren erhebliche Bauraumerfordernisse und auch Fehleranfälligkeiten, die sich aus der Lade- und Entladestrombelastung des Pufferkondensators oder entsprechenden Kondensatorblocks ergeben.

Davon ausgehend ist es Aufgabe der Erfindung, einen Generator mit einer Leistungsfaktor-Korrekturschaltung anzugeben, die einen verminderten Bauraum und eine erhöhte Zuverlässigkeit aufweist.

Diese Aufgabe wird mit dem Generator nach Anspruch 1 gelöst:

Der erfindungsgemäße Generator dient zum Betrieb chirurgischer Instrumente, d.h. zur Versorgung derselben mit typischerweise hochfrequenter Spannung und hochfrequentem Strom, wobei der Betrieb der Instrumente gepulst sein kann. Die Pulsung kann schon dadurch entstehen, dass das Instrument wiederholt sekundenweise ein- und wieder abgeschaltet wird, wie es der Chirurg im Rahmen seines Eingriffs vornimmt. Die Pulsung kann aber auch daraus resultieren, dass der zum Betrieb des Instruments gewählte Mode ein fortwährendes Ein- und Austasten der Spannung des HF-Generators erfordert. Diese Pulsung kann im Subhertzbereich oder auch im Bereich von einem oder wenigen Hertz liegen. Auch höherfrequente Pulsungen sind möglich.

Die Leistungsfaktor-Korrekturschaltung beruht wie üblich auf einer Sperrwandlerschaltung, deren Eingang mit einem Netzgleichrichter und deren Wandlerausgang mit mindestens einem Speicherkondensator verbunden ist. Außerdem weist der Sperrwandler einen elektronischen Schalter mit einer Steuerelektrode, beispielsweise einen FeldeffektTransistor mit einer Gate-Elektrode auf. Zur Steuerung des elektronischen Schalters dient eine Steuerschaltung, deren Schaltsignalausgang mit der Steuerelektrode, zum Beispiel dem Gate des Feldeffekt-Transistors, verbunden ist. Außerdem weist die Steuerschaltung einen Spannungsfühlereingang auf, der zum Beispiel über eine Spannungsabgriffsschaltung mit dem Wandlerausgang verbunden ist. So erhält die Steuerschaltung an ihrem Spannungsfühlereingang ein Signal, das die auf dem Speicherkondensator vorhandene Spannung kennzeichnet.

Die Steuerschaltung ist typischerweise eine integrierte Schaltung, die für den Betrieb von Leistungsfaktor-Korrekturschaltungen gestaltet, kommerziell hergestellt und in großem Umfang vertrieben wird und somit im Markt leicht erhältlich ist. Typischerweise sind solche Schaltungen jedoch nicht für Leistungsfaktor-Korrekturschaltungen geeignet, die abrupt und stark schwankende, insbesondere langsam im Hertz- oder Subhertzbereich gepulste Lasten versorgen oder dazu übergroße Kondensatorpakete erfordern würden. Die Erfindung hilft dem ab, indem zwischen der Spannungsabgriffsschaltung und dem Spannungsfühlereingang der Steuerschaltung ein Signalverstärker angeordnet ist. Damit wird die Steuerschaltung ohne inneren Eingriff zum Betrieb von Leistungsfaktor-Korrekturschaltungen tauglich, die stark wechselnde, insbesondere auch gepulste Lasten versorgen können.

Die Steuerschaltung ist vorzugsweise ein integrierter Schaltkreis, beispielsweise ein ICE3PCS01G von Infineon Technologies, ein L4985 von dem Hersteller STMicroelectronics, ein TEA2376DT von dem Hersteller NXP oder ein UCC28180 von Texas Instruments. Weitere ICs von diesen oder anderen Herstellern können ebenso Anwendung finden.

Diese Schaltkreise sind gemäß ihren Standardapplikationen zum Betrieb mit gleichmäßiger oder sich allmählich ändernder Belastung konzipiert und typisiert. Sie eignen sich zwar auch für schnell wechselnde Lasten, wobei aber bei schnellem Lastwechsel mit temporären Spannungsschwankungen am Wandlerausgang zu rechnen ist. Durch den erfindungsgemäß vorgesehenen zusätzlichen, dem Spannungsfühlereingang vorgeschalteten Verstärker, wird die Leistungsfaktor-Korrekturschaltung auch zum Betrieb schnell wechselnder Lasten bei gleichzeitiger Vermeidung größerer Spannungsschwankungen am Wandlerausgang tauglich, so dass der Generator mit dieser Leistungsfaktor-Korrekturschaltung auch Modes mit Pulsbetrieb bereitstellen kann. Dies ohne temporären Spannungsschwankungen mit einem vergrößerten Speicherkondensator(-paket) entgegenwirken zu müssen.

Steuerschaltungen der genannten Bauart können eine Überspannungs-Abschaltfunktion aufweisen, die dazu eingerichtet ist, den Sperrwandler abzuschalten, falls die Spannung an dem Spannungsfühlereingang VSENSE einen Grenzwert übersteigt. Zusätzlich kann die Steuerschaltung einen weiteren Signaleingang OVP aufweisen, der gegebenenfalls über eine Spannungsteilerschaltung mit dem Wandlerausgang verbunden ist, um diesen auf Überspannung zu überwachen. Um beim Hochlaufen des Sperrwandlers nach Start bei leerem Speicherkondensator, d.h. beim Einschalten bei noch ungeladenem Speicherkondensator, keine inkonsistenten Signale an den Spannungsfühlereingang VSENSE und den zusätzlichen Signaleingang OVP zu liefern, ist es zweckmäßig, den Verstärkungsfaktor des Verstärkers während des Betriebs der Leistungsfaktor-Korrekturschaltung variieren zu können. So kann vermieden werden, dass die Steuerschaltung in einen Fehlermodus schaltet und die Leistungsfaktor-Korrekturschaltung abschaltet.

Insbesondere kann der Verstärker dazu einen zur Steuerung der Verstärkung eingerichteten Steuereingang aufweisen. Über diesen ist der Verstärkungsfaktor des Verstärkers zwischen wenigstens zwei verschiedenen Werten umschaltbar. Vorzugsweise ist der erste Wert Eins und der zweite Wert größer als Eins. Dieses Konzept eignet sich insbesondere für ICs, bei denen eine Verstärkung des an den Spannungsfühlereingang VSENSE gelieferten Signals nicht vorgesehen ist. Bei solchen ICs kann es infolge der zusätzlichen Signalverstärkung sonst zu inkonsistenten Signalen an den verschiedenen Eingängen des ICs kommen. Dies kann z.B. beim Hochlaufen der Schaltung, insbesondere beim Kaltstart, zu Schwierigkeiten, wie z.B. zur Fehlerabschaltung führen.

Bei einer bevorzugten Ausführungsform weist die Steuerschaltung einen Signalausgang VB_OK auf, der kennzeichnet, dass an dem Wandlerausgang der Leistungsfaktor-Korrekturschaltung die gewünschte Sollspannung anliegt. Dieser Signalausgang VB_OK ist vorzugsweise mit dem Schalteingang des Verstärkers verbunden. Damit wird erreicht, dass die Leistungsfaktor-Korrekturschaltung nach dem Einschalten an ihrem Spannungsfühlereingang ein unverstärktes Signal erhält und somit den Sperrwandler gemäß ihrer Spezifikation steuert. Ist die Sollspannung am Wandlerausgang von beispielsweise 400 Volt erreicht, wechselt das an dem Signalausgang VB_OK der Steuerschaltung anliegende Signal seinen Wert. Dieses Signal wird als Schaltsignal an den Steuereingang des Verstärkers geleitet, wodurch dieser nun einen Verstärkungsfaktor aufweist, der dann größer als Eins ist. Damit wird die Schleifenverstärkung in einer durch den Verstärker und die Steuerschaltung gebildeten Regelschleife erhöht, was, nachdem die Sollspannung (zum Beispiel 400 Volt) einmal erreicht worden ist, nun zu einer verbesserten Regelgenauigkeit führt. Damit wird es möglich, den zur Pufferung von Lastschwankungen am Wandlerausgang vorgesehenen Kondensators oder Kondensatorpakets zu verkleinern, was Bauraum spart und wegen der verminderten Lade- und Entladeströme auch eine Erhöhung der Zuverlässigkeit des Generators insgesamt ermöglicht. Andererseits wird eine Störung der Arbeit des ICs, insbesondere in der Hochlaufphase, vermieden.

Weitere Details und Vorzüge der vorliegenden Erfindung ergeben sich aus der Zeichnung sowie der Beschreibung der zugehörigen Figuren sowie Ansprüchen. Es zeigen:
Figur 1 Einen Generator zum Betrieb chirurgischer Instrumente mit Darstellung seiner Funktionsblöcke,
Figur 2 den Netzgleichrichter und die Leistungsfaktor-Korrekturschaltung PFC nebst Steuerschaltung des Generators nach Figur 1 als Prinzip-Schaltbild,
Figur 3 den Steuerbahnverstärker der Leistungsfaktor-Korrekturschaltung nach Figur 2 als Prinzip-Schaltbild,
Figur 4 einen Schaltungszweig des Steuerbahnverstärkers nach Figur 3,
Figur 5 eine zu der Leistungsfaktor-Korrekturschaltung nach Figur 2 gehörige Einschalt-Verzögerungsschaltung und
Figur 6 ein Diagramm zur Veranschaulichung des Hochlaufbetriebs und des gepulsten Betriebs der Leistungsfaktor-Korrekturschaltung.

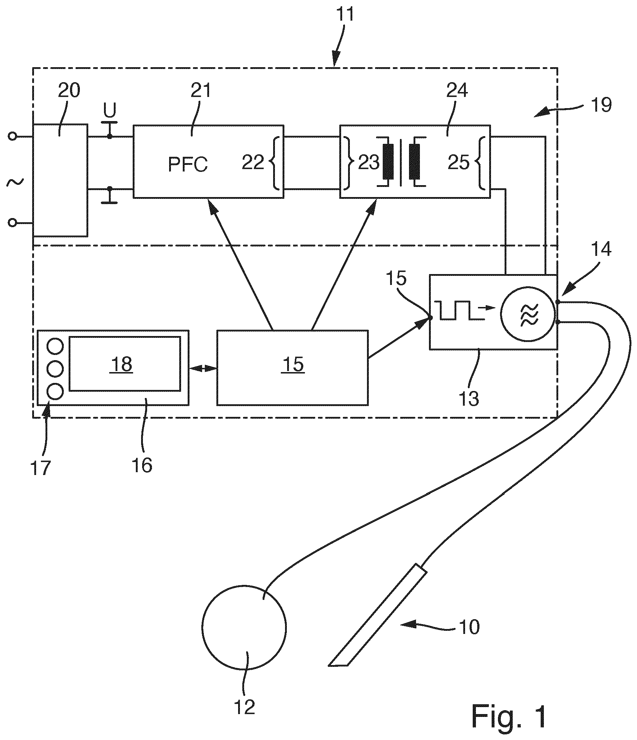

In Figur 1 sind ein medizinisches Instrument 10 zur chirurgischen oder anderweitigen Einwirkung auf einen Patienten und ein zur Speisung des Instruments 10 dienender Generator 11 veranschaulicht. Das Instrument 10 ist als monopolares Instrument veranschaulicht, dem eine am Patienten zu befestigende Neutralelektrode 12 zugeordnet ist. Die Neutralelektrode 12 und das Instrument 10 sind mit dem Generator 11 über elektrische Leitungen verbunden. Anstelle eines monopolaren Instruments können jedoch auch bipolare oder mehrpolare Instrumente Anwendung finden, die dann gegebenenfalls auch ohne Neutralelektrode auskommen.

Der Generator 11 ist insbesondere dazu eingerichtet und dafür geeignet, Instrumente in Modes zu betreiben, in denen die durch das Instrument dargestellte elektrische Last und somit die von dem Instrument aufgenommene elektrische Leistung sprunghaft zwischen sehr geringen Werten und sehr hohen Werten wechselt. Bei den geringen Werten kann es sich um Werte nahe Null Watt oder von lediglich wenigen Watt elektrischer Leistung handeln. Bei den hohen Werten kann es sich um Leistungen von mehreren 100 Watt bis in den Kilowattbereich hinein handeln.

Der Generator 11 enthält einen Hochfrequenzoszillator 13, der darauf eingerichtet ist, die zum Betrieb des Instruments 10 erforderliche elektrische Leistung an einem Ausgang 14 bereitzustellen, an den das Instrument 10 und die Neutralelektrode 12 angeschlossen sind. Außerdem weist der Hochfrequenzoszillator 13 einen Steuereingang 15 auf, der dazu eingerichtet ist, Steuerimpulse zu empfangen, die den Hochfrequenzoszillator 13 steuern. Beispielsweise können die Steuerimpulse das Einschalten und das Ausschalten des Hochfrequenzoszillators oder auch eine anderweitige Leistungsmodulation desselben bewirken. In Figur 1 ist in dem den Hochfrequenzoszillator 13 kennzeichnenden Block als Beispiel für den zeitlichen Verlauf eines Steuerimpulses eine Rechteckwelle veranschaulicht, gemäß derer der Hochfrequenzoszillator 13 in gegebenen Zeitabständen ein- und ausgeschaltet wird. Dabei können die Abstände zwischen Ein- und Ausschaltzeitpunkten, d.h. zwischen Vorder- und Rückflanke eines Impulses einer Rechteckwelle, einige 10 bis einige 100 Millisekunden oder auch eine oder mehrere Sekunden betragen. Mit anderen Worten, die Frequenz dieser Steuerimpulse kann im Subhertz- oder im Hertzbereich liegen.

Zur Erzeugung der Steuerimpulse und somit zur Festlegung des Modes, in welchem der Generator 11 insgesamt und insbesondere der Hochfrequenzoszillator 13 arbeiten, dient eine Systemsteuerung 15. Diese ist mit einer Kommunikationseinheit 16 verbunden, die dazu eingerichtet ist, Nutzereingaben zu empfangen und Ausgaben anzuzeigen. Dazu weist die Kommunikationseinheit 16 Eingabeelemente 17 z.B. in Gestalt von Tasten, Knöpfen oder Schaltern sowie eine Ausgabeeinheit 18 z.B. in Gestalt eines oder mehrerer Bildschirme und/oder Anzeigeinstrumente und/oder Kontrolllampen und dergleichen auf.

Zur Stromversorgung sowohl insbesondere des Hochfrequenzoszillators 13 aber auch der Systemsteuerung 15 und der Kommunikationseinheit 16 dient eine im oberen Teil des Generators 11 veranschaulichte Stromversorgungseinheit 19. Die Stromversorgungseinheit 19 ist eingangsseitig an das öffentliche Stromversorgungsnetz angeschlossen und versorgt die Komponenten des Generators 11 mit der gebotenen elektrischen Sicherheit potenzialgetrennt mit Strom. Dies bedeutet, dass die Stromversorgungseinheit 19 zwischen der Netzseite und der Patientenseite potenzialtrennend ausgebildet ist, sodass auch Potenzialdifferenzen von mehreren tausend Volt zwischen dem Stromversorgungsnetz einerseits und dem Patienten bzw. dem Instrument 10 und der Neutralelektrode andererseits nicht zur schädlichen elektrischen Durchströmung des Patienten führen.

Zu der Stromversorgungseinheit 19 gehört zunächst ein an das elektrische Netz angeschlossener Eingangsgleichrichter 20 mit Netzfilter. Der Eingangsgleichrichter 20 ist typischerweise als Graetzbrücke ausgebildet und liefert an seinem Ausgang eine wellige Gleichspannung Uᵣ, die einem Eingang der Leistungsfaktorkorrekturschaltung 21 zugeführt wird. Die Leistungsfaktorkorrekturschaltung 21 (PFC) setzt diese Spannung in eine an ihrem Ausgang anstehende Gleichspannung um, die höher ist, als die Spitzenspannung der Netzspannung.

Die Leistungsfaktorkorrekturschaltung 21 weist einen Ausgang 22 auf, an dem die von der Leistungsfaktorkorrekturschaltung 21 umgesetzte Gleichspannung ansteht. Der Ausgang 22 ist an den Eingang 23 eines potentialtrennenden Spannungswandlers 24 angeschlossen, dessen Ausgang 25 wiederum mit dem Hochfrequenzoszillator 13 verbunden ist, um diesen mit elektrischer Leistung zu speisen. Der Spannungswandler 24 ist potentialtrennend ausgebildet, d.h. der Eingang 23 und der Ausgang 25 sind galvanisch getrennt. Die Isolationsfestigkeit dieser galvanischen Trennung liegt typischerweise im Bereich oberhalb von 6 kV, besser 10 kV oder 12 kV.

Optional kann eine steuernde Verbindung zum Beispiel in Gestalt einer Datenverbindung zwischen der Systemsteuerung 15 und dem Spannungswandler 24 vorgesehen sein. Diese Datenverbindung kann beispielsweise dazu dienen, die Größe der an dem Ausgang 25 abgegebenen Spannung oder andere Parameter vorzugeben. Ebenso kann optional eine steuernde Verbindung zwischen der Systemsteuerung 15 und der Leistungsfaktor-Korrekturschaltung 21 vorgesehen sein, beispielsweise um die Leistungsfaktor-Korrekturschaltung 21 zu aktivieren oder zu deaktivieren, beispielsweise um einen Standby-Modus vorzugeben.

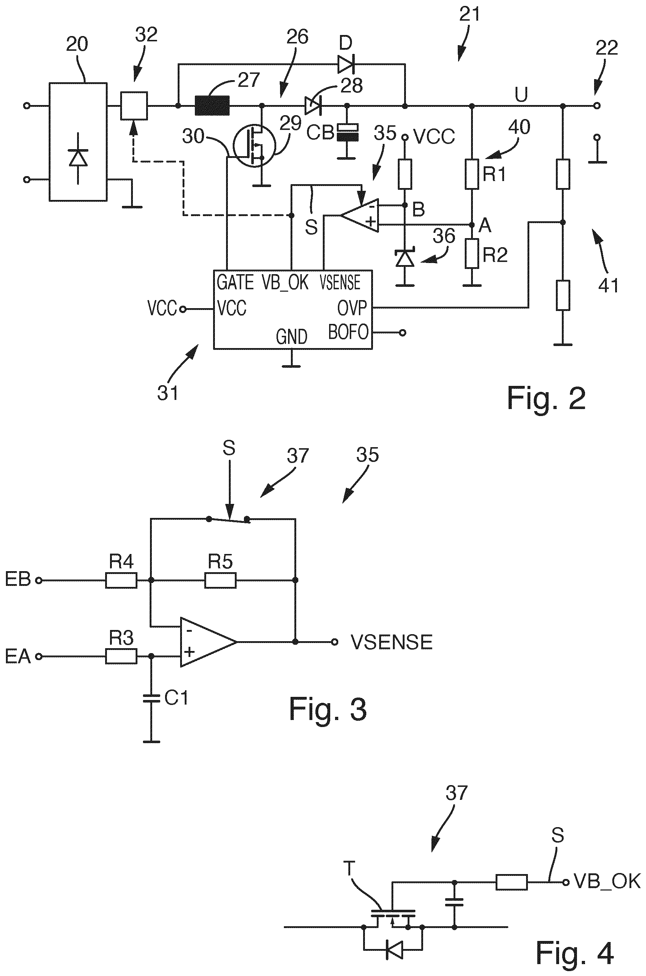

Ein Hauptaugenmerk der Erfindung liegt auf der Ausbildung der Leistungsfaktor-Korrekturschaltung 21, die in Figur 2 in Form eines Prinzipschaltbilds wiedergegeben ist. Die Leistungsfaktor-Korrekturschaltung 21 enthält eine Sperrwandlerschaltung 26, deren Hauptbestandteile ein Induktor 27, eine mit diesem in Flussrichtung in Reihe geschaltete Diode 28, ein dazwischen angeschlossener, gegen Masse führender elektronischer Schalter 29 und ein an die Diode angeschlossener, ebenfalls auf Masse bezogener Speicherkondensator CB sind. Der gesteuerte Schalter 29 ist vorzugsweise ein Feldeffekttransistor, dessen Source mit Masse und dessen Drain mit dem Verbindungspunkt zwischen dem Induktor 27 und der Diode 28 verbunden ist. Seine Steuerelektrode 30 (im Falle eines Feldeffekttransistors sein Gate) ist mit einer Steuerschaltung 31 verbunden, die als integrierte Schaltung ausgebildet ist. Als bevorzugter Baustein für die Steuerschaltung 31 ist der Schaltkreis ICE3PCS01G von dem Hersteller Infineon Technologies vorgesehen. Im Markt sind weitere geeignete, integrierte Schaltkreise vorhanden, die hier Anwendung finden können, beispielsweise L4985 von ST Microelectronics, TEA2376DT von NXP und UCC28180 von TXP und viele andere mehr.

Die Sperrwandlerschaltung 26 entspricht bis auf die nachstehend beschriebenen Besonderheiten weitgehend der dem Datenblatt zu der Steuerschaltung 31 zu entnehmenden Standardschaltung. Für das Verständnis der Schaltung hier nicht zwingend notwendige Anschlüsse der Steuerschaltung 31 und deren Beschaltung sind in Figur 2 weggelassen. Die Anschlüsse sind gleichwohl vorhanden und können wie vom Datenblatt vorgegeben beschaltet sein.

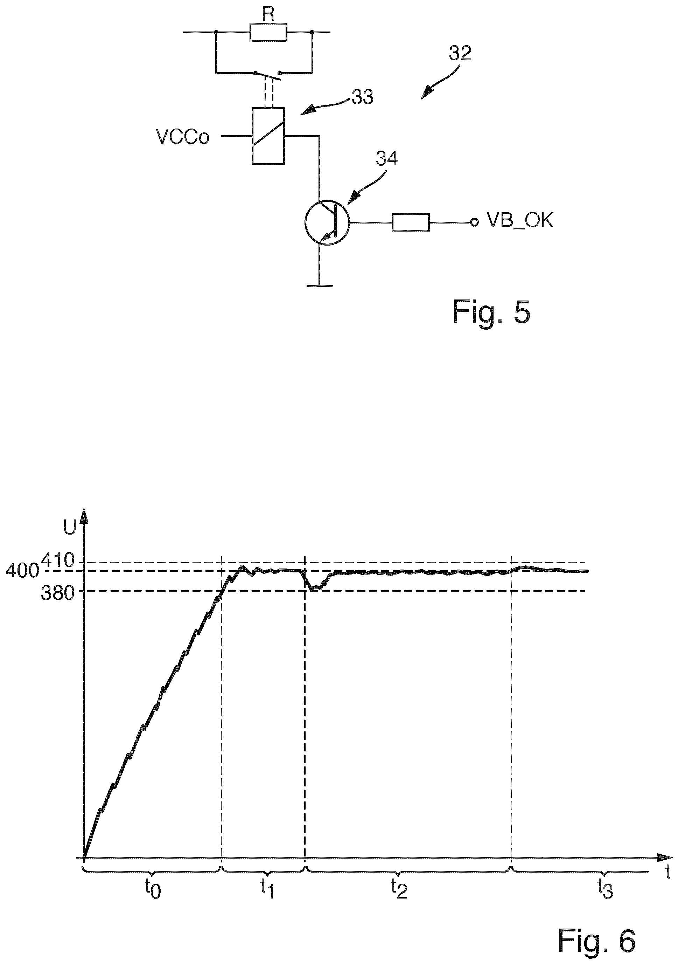

Zwischen dem Eingangsgleichrichter 20 und der Sperrwandlerschaltung 26 ist eine Einschaltstrombegrenzungsschaltung 32 vorgesehen, die in Figur 5 gesondert veranschaulicht ist. Es handelt sich bei der Einschaltstrombegrenzungsschaltung 32 letztendlich um einen Strombegrenzungswiderstand R, der durch den Schaltkontakt eines Relais 33 überbrückt wird, sobald ein entsprechender Anschluss VB_OK der Steuerschaltung 21 auf ein positives, von Null verschiedenes Potential wechselt, um den angeschlossenen Transistor 34 leitend zu machen und dabei den Kontakt des Relais 33 zu schließen. An dem Anschluss VB_OK liegt eine positive von Null verschiedener Spannung an, sobald die Spannung am Wandlerausgang 22 einen Sollbereich erreicht. Dieser liegt im vorliegenden Fall entsprechend der Dimensionierung einer Spannungsabgriffsschaltung 40 zwischen 380 und 410 Volt, bei einer gewünschten Wandlerausgangsspannung U von 400 Volt. Die Spanungsabgriffsschaltung 40 ist eine Spannungsteilerschaltung mit zwei oder mehreren ohmschen Widerständen R1, R2.

Die Steuerschaltung 31 weist außerdem einen Überspannungsschutzeingang OVP auf, der über einen Spannungsteiler 41 an den Wandlerausgang 22 angeschlossen ist. Der Spannungsteiler ist dabei so dimensioniert, dass eine Abschaltgrenze an dem Überspannungsschutzeingang OVP erst dann erreicht wird, wenn an dem Wandlerausgang 22 eine nicht hinnehmbare Überspannung erkannt wird. Diese ist beispielsweise durch die Spannungsfestigkeit des Speicherkondensators CB festgelegt und kann zum Beispiel 420 Volt betragen.

Die Besonderheit der Sperrwandlerschaltung 26 besteht gegenüber der standardgemäßen Anwendung der Steuerschaltung 31 darin, dass zwischen dem Spannungsfühlereingang VSENSE und der Spannungsabgriffsschaltung 40 ein Verstärker angeordnet ist. Der Verstärker 35 weist einen nichtinvertierenden Eingang auf, der mit dem Spannungsabgriffspunkt A des aus den Widerständen R1 und R2 bestehenden der Spannungsabgriffsschaltung 40 verbunden ist. Der Verstärkerausgang ist hingegen mit dem Spannungsfühlereingang VSENSE verbunden.

Bei einer bevorzugten Ausführungsform weist der Verstärker 35 außerdem einen invertierenden Eingang auf, der mit einer Referenzspannung verbunden ist. Diese kann an einem Spannungsnormal, beispielsweise in Gestalt einer Referenzspannungsquelle 36, z.B. einer Z-Diode, über einen Vorwiderstand aus der Versorgungsspannung VCC (zum Beispiel 12 Volt) erzeugt werden. Die Referenzspannungsquelle 36 ist darauf eingerichtet, diejenige Spannung bereitzustellen, die auch an dem Spannungsabgriffspunkt A anliegt, wenn die Spannung U ihren Sollwert eingenommen hat. In diesem Fall ist die Differenz zwischen der Spannung an dem Spannungsabgriffspunkt A und der Referenzspannung gleich Null. Außerdem stimmt die Referenzspannung mit derjenigen Spannung überein, die an dem Eingang VSENSE der Steuerschaltung (insbesondere ICE3PCS01G von Infineon) anstehen muss, wenn die Spannung U an dem Ausgang 22 ihren Sollwert eingenommen hat. Diese Spannung der Referenzspannungsquelle 36 VSENSE führt weder zu einer Erhöhung noch zu einer Erniedrigung der Spannung U, wenn sie an dem Eingang VSENSE anliegt. Im vorliegenden Ausführungsbeispiel wird als Referenzspannungsquelle von 2,5 V genutzt, z.B. in Gestalt der ADR5041BKSZ von Analog Devices Inc.

Der Verstärker 35 kann als Verstärker mit umschaltbarem Verstärkungsfaktor ausgebildet sein, wie es in Figur 3 veranschaulicht ist. Bei dem Verstärker 35 kann es sich um einen Operationsverstärker handeln, dessen nichtinvertierender Eingang über einen Widerstand R3 mit dem Verstärkereingang EA verbunden ist. Der invertierende Eingang ist hingegen über einen in seiner Größe mit dem Widerstand R3 übereinstimmenden Widerstand R4 mit dem Verstärkereingang EB verbunden. Im Rückkopplungszweig zwischen dem Ausgang des Operationsverstärkers und dem Widerstand R4 ist ein Widerstand R5 angeordnet, dessen Verhältnis zu dem Widerstand R4 die Größe des Verstärkungsfaktors bestimmt. Parallel zu dem Widerstand R5 kann ein Schalter 37, insbesondere ein elektronischer Schalter, vorgesehen sein, mit dem der Widerstand R5 überbrückbar ist. Zur Steuerung des Schalters 37 kann ein Signal S dienen, mit dem der Schalter 37 gezielt geöffnet oder geschlossen werden kann.

Figur 4 veranschaulicht eine Realisierung des Schalters 37 mittels eines Feldeffekttransistors T, dessen Gate gegebenenfalls über einen Widerstand mit dem Anschluss VB_OK der Steuerschaltung 31 verbunden ist.

Der Verstärkungsfaktor des Verstärkers 35 ist bei geschlossenem, d.h. stromdurchlässigem Schalter 37 "Eins". Ist der Schalter 37 hingegen geöffnet, d.h. stromundurchlässig, wird der Verstärkungsfaktor durch das Verhältnis der Widerstände R4 und R5 zueinander bestimmt. Sind diese Widerstände gleich groß, ist der Verstärkungsfaktor "Zwei", wie es im vorliegenden Fall bevorzugt wird. Es ist aber auch möglich, andere Verstärkungsfaktoren vorzusehen.

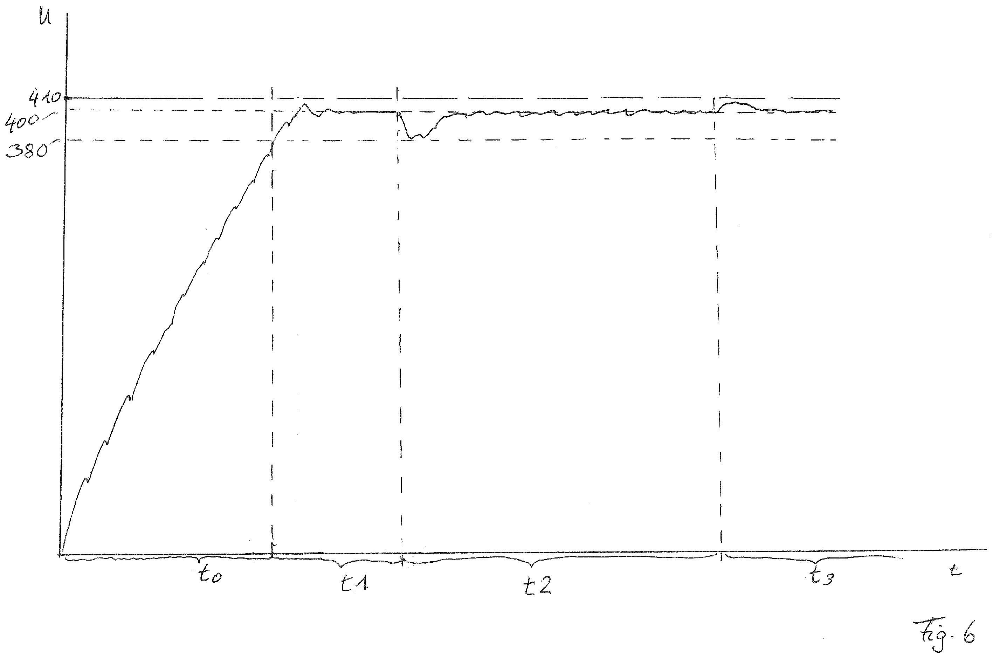

Der insoweit beschriebene Generator 11 arbeitet wie folgt:
Nach dem Einschalten des Generators muss die Leistungsfaktor-Korrekturschaltung 21 zunächst den Speicherkondensator CB aufladen. Dazu kann Ladestrom über die Diode D fließen, wobei der Ladestrom durch die Einschaltstrombegrenzung 32 zunächst begrenzt wird. Das Signal S an dem Anschluss VB_OK ist Null, womit angezeigt wird, dass die Spannung an dem Wandlerausgang 22 noch außerhalb des Sollspannungsbereichs liegt. Weil der Schalter 37 in diesem Zustand geschlossen ist, hat der Verstärker 35 die Verstärkung von Eins. Mit anderen Worten, an dem Eingang VSENSE der Steuerschaltung 31 liegt die Referenzspannung der Referenzspannungsquelle 36, je nach Vorzeichen vermehrt oder vermindert um die Differenz zwischen der Spannung an dem Spannungsabgriffspunkt A und der Referenzspannung, an. Die Sperrwandlerschaltung 26 arbeitet somit auf herkömmliche Weise mit der von der Steuerschaltung 31 intern vorgegebenen Regelverstärkung des in Figur 6 angegebenen Zeitraums t0.

Sobald die auf dem Speicherkondensator CB und somit auch an dem Wandlerausgang 22 anstehende Spannung einen unteren Grenzwert eines Spannungstoleranzbandes erreicht hat, geht der das Erreichen der Sollspannung anzeigende Ausgang VB_OK auf einen positiven von Null verschiedenen Wert. Durch geeignete Bemessung der durch einen Spannungsteiler gebildeten Spannungsabgriffsschaltung 40 oder des Spannungsteilers 41 an dem Anschluss OVP kann dieser Grenzwert geeignet festgelegt werden, beispielsweise auf 380 Volt.

Indem das Signal S mit Erreichen der so festgelegten Schaltschwelle auf einen positiven Wert geht, wird einerseits die Eingangsstrombegrenzung 32 durch Kurzschluss des Widerstands R inaktiviert und andererseits der Schalter 37 geöffnet. Dadurch wird nun die Referenzspannung, vermehrt oder vermindert um die zweifach verstärkte Differenz zwischen den Spannungen an den Spannungsabgriffspunkten A und B (Figur 2) an den Spannungsfühlereingang VSENSE gegeben. Die Spannungsregelung der Steuerschaltung 31 und die durch sie gebildete Regelschleife arbeitet nun mit erhöhter Verstärkung. Dies, sobald und solange die Spannung U an dem Wandlerausgang innerhalb des festgelegten Toleranzbands wie beispielsweise gemäß Figur 6 zwischen 380 und 410 Volt liegt. Ist die durch einen integrierten Schaltkreis gebildete Steuerschaltung 31 als Proportionalregler (P-Regler), Proportional-Integral-Regler (PI-Regler) oder Proportional-Integral-Differenzial-Regler (PID-Regler) konzipiert, wird mit der Erfindung der Proportionalanteil P des Reglers wenigstens dann (und vorzugsweise nur dann) erhöht, wenn die Spannung U an dem Ausgang 22 in einem vorgegebenen Toleranzband liegt. Die kann durch ein Signal an einem Ausgang VB-OK der integrierten Steuerschaltung 31 angezeigt sein.

Mit der erhöhten Verstärkung des Verstärkers 35 wird nun die gewünschte Sollspannung von 400 Volt geregelt, wobei zum Beispiel lastsprungbedingte temporäre Abweichungen durch die erhöhte Verstärkung minimiert werden. Dies veranschaulicht ein Vergleich der auf das Einschalten folgenden Zeitabschnitte t1, t2 und t3 in Figur 6. In dem Zeitraum t1 wird an dem Wandlerausgang 22 wenig Strom abgenommen. Nach einem Einschwingen der Spannung auf die Sollspannung von 400 Volt bleibt die Spannung konstant. Zu Beginn des nachfolgenden Zeitabschnitts t2, der zum Beispiel 100 ms, 200 ms oder mehrere 100 ms betragen kann, wird an dem Wandlerausgang 22 eine hohe Leistung, z.B. die Maximalleistung abgenommen. Der Übergang zwischen niedriger und hoher Leistung kann sehr schnell, z.B. in lediglich wenigen Millisekunden oder in Bruchteilen derselben erfolgen. Die Spannung bricht dabei kurzzeitig ein, um dann sehr schnell wieder auf den Sollwert von 400 Volt eingeregelt zu werden. Die sichtbare geringe Restwelligkeit geht auf die Netzwelligkeit, nicht aber auf Regeleffekte zurück.

Durch die Verstärkung des zwischen den Spannungsabgriffspunkten A und B abgenommenen Signals ist der Spannungseinbruch der Spannung U an dem Ausgang 22 signifikant kleiner, als er ohne diese zusätzliche Verstärkung bei gleich dimensioniertem Speicherkondensator CB wäre. Auch der Spannungsanstieg nach dem Ende der Lastperiode t2 ist signifikant geringer, als er ohne den Verstärker 35 wäre. Deswegen lassen sich mit der insoweit beschriebenen Leistungsfaktor-Korrekturschaltung 21 Generatoren 11 realisieren, deren Hochfrequenzoszillator 13 sehr stark und kurzfristig sogar zwischen Null- und Volllast wechselnde Arbeitsperioden aufweisen, ohne dass dazu der Speicherkondensator CB größer als üblich dimensioniert werden müsste. Der Kondensator CB kann auf denjenigen Wert dimensioniert werden, den er bei der geforderten Restwelligkeit und der geforderten Volllast ohne Berücksichtigung der hier in Betracht gezogenen Lastsprünge lediglich haben müsste.

Der Verstärker 35 arbeitet mit einer Verstärkung größer Eins, sobald und solange das Signal an dem Anschluss VBOK von Null verschieden ist, d.h., sobald und solange die Spannung U an dem Wandlerausgang 22 innerhalb des gewünschten Toleranzbereichs liegt. Außerhalb dieses Toleranzbereichs arbeitet der Verstärker 35 mit einem Verstärkungsfaktor von Eins, das heißt ohne Verstärkung.

Ein erfindungsgemäßer für gepulsten Betrieb geeigneter Generator 11 weist eine Leistungsfaktor-Korrekturschaltung 21 mit einer integrierten Steuerschaltung 31 auf. Diese enthält einen Rückkopplungsweg, über den eine Regelschleife gebildet ist. Die Leistungsfaktor-Korrekturschaltung 21 ist dazu eingerichtet, den Schalter 29 mittels Schaltimpulsen so zu steuern, dass Spannungsschwankungen an dem Spannungsfühlereingang VSENSE entgegengewirkt wird.

Innerhalb des zur Spannungsregelung vorgesehenen Rückkopplungswegs ist der Verstärker 35 angeordnet, der einen Verstärkungsfaktor größer als 1 aufweist. Dazu ist dem Spannungsfühlereingang (VSENSE) der Steuerschaltung 31 ein Verstärker 35 vorgeschaltet. Damit lassen sich Spannungsschwankungen an dem Wandlerausgang 22 minimieren, die in Folge von Lastsprüngen auftreten. Es ist möglich, den Verstärker 35 so auszubilden, dass sein Verstärkungsfaktor in einem ersten Zustand den Wert 1 (oder einen anderen festen Wert) und in einem zweiten Zustand einen davon verschiedenen, vorzugsweise größeren Wert einnimmt. Weiter ist es möglich, die Verstärkung lediglich dann auf den Wert von größer als 1 zu setzen, wenn die Spannung an dem Wandlerausgang innerhalb eines Toleranzbereichs liegt, der für den gewöhnlichen Betrieb der Leistungsfaktor-Korrekturschaltung 21 vorgesehen ist. Außerhalb dieses Toleranzbereichs ist die Verstärkung des Verstärkers 35 dann genau 1. Damit wird der gewöhnliche Betrieb der integrierten Steuerschaltung 31 insbesondere beim Hochlaufen nicht gestört.

### Bezugszeichen:

- 10: Instrument
- 11: Generator
- 12: Neutralelektrode
- 13: Hochfrequenzoszillator
- 14: Ausgang
- 15: Systemsteuerung
- 16: Kommunikationseinheit
- 17: Eingabeelemente
- 18: Anzeigeelemente
- 19: Stromversorgungseinheit
- 20: Eingangsgleichrichter
- Ur: Spannung am Ausgang des Eingangsgleichrichter 20
- 21: Leistungsfaktor-Korrekturschaltung
- 22: Wandlerausgang
- 23: Eingang des Spannungswandlers 24
- 24: Spannungswandler
- 25: Ausgang
- 26: Sperrwandlerschaltung
- 27: Induktor
- 28: Diode
- 29: gesteuerter Schalter / Transistor
- 30: Steuerelektrode
- 31: Steuerschaltung
- 32: Einschaltstrombegrenzungsschaltung
- R: Strombegrenzungswiderstand
- 33: Relais
- VB_OK: Anschluss zur Anzeige der Sollspannung
- 34: Transistor
- U: Wandlerausgangsspannung
- OVP: Überspannungsschutzeingang
- VSENSE: Spannungsfühlereingang
- 35: Verstärker
- 36: Referenzspannungsquelle
- VCC: Versorgungsspannung
- 37: Schalter
- T: Feldeffekttransistor
- 40: Spannungsabgriffsschaltung
- 41: Spannungsteiler

## Patentansprüche

1. Generator (11) zum Betrieb chirurgischer Instrumente (10), insbesondere in Pulsbetrieb,
mit einer Leistungsfaktorkorrekturschaltung (21), die eine Sperrwandlerschaltung (26) enthält, deren Eingang mit einem Netzgleichrichter (20) und deren Wandlerausgang (22) mit mindestens einem Speicherkondensator (CB) verbunden ist und die einen elektronischen Schalter (29) mit einer Steuerelektrode (30) aufweist,
mit einer Spannungsabgriffsschaltung (40), die an den Wandlerausgang (22) angeschlossen ist und die einen Spannungsabgriffspunkt (A) aufweist,
mit einer Steuerschaltung (31), die einen mit der Steuerelektrode (30) verbundenen Schaltsignalausgang (GATE) und einen Spannungsfühlereingang (VSENSE) aufweist, der zum Empfang eines Signals eingerichtet ist, das die auf dem Speicherkondensator (CB) vorhandene Spannung kennzeichnet,
mit einem Verstärker (35), der einen mit dem Spannungsabgriffspunkt (A) verbundenen Verstärkereingang (EA) und einen Verstärkerausgang aufweist, der mit dem Spannungsfühlereingang (VSENSE) der Steuerschaltung (31) verbunden ist.

2. Generator nach Anspruch 1, **dadurch gekennzeichnet, dass** die Steuerschaltung (31) ein integrierter Schaltkreis ist, der einen intern festgelegten Zusammenhang zwischen dem Signal an dem Spannungsfühlereingang (VSENSE) und den von ihm abgegebenen Schaltimpulsen an dem Schaltsignalausgang (GATE) aufweist.

3. Generator nach Anspruch 2, **dadurch gekennzeichnet, dass** der integrierte Schaltkreis für Anwendungszwecke mit ungepulsten Lasten typisiert ist.

4. Generator nach einem der vorstehenden Ansprüche, **dadurch gekennzeichnet, dass** die Steuerschaltung (31) einen Signalausgang (VB_OK) aufweist, der dazu eingerichtet ist, ein Signal abzugeben, dass signalisiert, ob die an dem Wandlerausgang (22) messbare Spannung (U) in einem gegebenen Toleranzbereich liegt.

5. Generator nach einem der vorstehenden Ansprüche, **dadurch gekennzeichnet, dass** die Steuerschaltung (31) einen Signaleingang (OVP) aufweist, der zur Erfassung einer Überspannung am Wandlerausgang (22) eingerichtet ist.

6. Generator nach Anspruch 5, **dadurch gekennzeichnet, dass** die Steuerschaltung (31) dazu eingerichtet ist, die an dem Spannungsfühlereingang (VSENSE) und die an dem Signaleingang (OVP) anliegenden Spannungen zu vergleichen und die Sperrwandlerschaltung (26) stillzusetzen, wenn die Differenz zwischen ihnen einen Grenzwert übersteigt.

7. Generator nach einem der vorstehenden Ansprüche, **dadurch gekennzeichnet, dass** der Verstärker (35) ein Differenzverstärker mit einem an eine Referenzspannungsquelle (36) angeschlossenen negierenden und einem an den Spannungsabgriffspunkt (A) angeschlossenen nichtnegierenden Eingang ist.

8. Generator nach einem der vorstehenden Ansprüche, **dadurch gekennzeichnet, dass** der Verstärker (35) einen zur Steuerung der Verstärkung eingerichteten Eingang (S) aufweist.

9. Generator nach Anspruch 8, **dadurch gekennzeichnet, dass** der Eingang (S) ein Schalteingang ist, der dazu eingerichtet ist, ein Schaltsignal zu empfangen, um den Verstärkungsfaktor des Verstärkers (35) zwischen einem ersten Wert und einem zweiten Wert umzuschalten.

10. Generator nach Anspruch 8, **dadurch gekennzeichnet, dass** der erste Wert Eins beträgt und der zweite Wert größer als Eins ist.
